# EUROPEAN PATENT APPLICATION

(11) **EP 4 775 166 A1**
(43) Date of publication of application: **15.07.2026**
(21) Application number: 25151793.4
(22) Date of filing: 14.01.2025
(51) Int. Cl.: A61B 34/10, A61B 18/12, G06T 19/00, A61B 18/00, A61B 18/14

(54) **THERMAL ABLATION TRAJECTORY CALCULATION**

(71) Applicant: Siemens Healthineers AG, 91301 Forchheim (DE)
(72) Inventor: Audigier, Chloé, 3072 Ostermundigen (CH); Frings, Oliver, 91058 Erlangen (DE); Joy, Vaisakh Nappady, 91052 Erlangen (DE); Kellnberger, Stephan, 85604 Zorneding (DE); Meister, Felix, 91054 Erlangen (DE)
(74) Representative: Siemens Healthineers Patent Attorneys

(57) **Abstract**

The present invention relates to a method, device and system for calculating a trajectory for an ablation electrode for tissue ablation during an interventional procedure. The method may comprise:
- Receiving (S1) interventional image data;
- Executing a segmentation algorithm (S2) on the received interventional image data for providing a set of segmentation masks, comprising at least a tumor mask and a body mask;
- Determining (S3) a set of target points, based on the provided tumor mask;
- Determining (S4) a set of skin entry points, based on the provided body mask;
- Computing (S5) a set of trajectory candidates, wherein a trajectory candidate comprises at least a pair of target point and skin entry point;
- Receiving (S6) a set of pre-definable constraints;
- Classifying (S7) the received set of constraints in at least two classes: a first type constraint which are to be met mandatorily and a second type constraints which need not to be met mandatorily;
- Defining (S8) differently sized and ordered subsets of trajectory candidates, wherein the subsets differ in conforming with the second type of constraints,
- Executing an optimization algorithm (S9) on the defined subsets for calculating (S10) at least one trajectory as result, starting with an initial subset, conforming with all first type constraints and with all second type constraints by computing a cost value, based on a cost function, and if no solution with 100% tumor coverage is found: iteratively executing the optimization algorithm for the next subset within the ordered subsets, so that the second type constraints are stepwise relaxed.

## Description

Independent of the grammatical term usage, individuals with male, female or other gender identities are included within the term.

The present invention is in the field of medical surgery, in particular thermal ablation and relates to a computer-implemented method and system for calculating a trajectory for an ablation electrode, like a needle, used in tissue ablation during an interventional procedure. This technology is in the field of medical imaging and surgical planning and procedure.

Liver cancer is the fourth highest occurring cancer type in the world. It can be treated invasively with resection or transplantation or minimally invasively with thermal ablation. For example, radiofrequency ablation is a widely used technique.

Tissue ablation is a minimally invasive surgical method that involves destroying abnormal tissues in the body, like cancer structures. This can be achieved through various methods such as radiofrequency, laser, microwave, ultrasound, or cryoablation. The procedure is often guided by imaging techniques to accurately target the abnormal tissue and avoid damaging healthy surrounding tissues.

Automatic planning algorithms for thermal ablation procedures have received significant research attention in recent years, because they could provide solutions much faster and much more reproducible compared to the manual planning approach. To find a solution to the multi-constraint multi-objective combinatorial optimization, planning algorithms mainly rely on conventional optimization methods (e.g., the Nelder-Mead simplex algorithm [7], quasi-exhaustive search [8], or solvers for the minimum set cover problem to satisfy the Pareto optimality [5]).

These algorithms, however, require a precise cost function that reflects the clinical constraints and prioritizes them to compute only a single solution. Alternatively, they allow to query solutions on the Pareto front by having the operator interact with the software.

Recently, the use of deep reinforcement learning has been explored. Specifically, a deep-learning-based agent learns to optimally place the thermal ablation needle(s) by interacting with a virtual patient environment and receiving positive or negative rewards depending on whether it optimizes the multiple constraints or disobeys them [9].

Whether it is conventional or Al-based planning, these methods commonly do not account for the execution complexity of a computed ablation needle trajectory. Consequently, these algorithms may return an ablation needle plan that is optimal for the considered constraints, but not clinically relevant since it is too difficult or too risky to execute. In such a scenario, the clinician may have to re-run the algorithm multiple times (each time with changed input parameters) until a more feasible solution is found. Alternatively, the clinician may manually select a different solution from the computed Pareto front if it was computed.

The trajectory of the ablation electrode is a critical aspect of the procedure, as it determines the path the electrode will take from the skin entry point to the target point within the tumor. This trajectory needs to be carefully planned to ensure the ablation procedure is effective and minimizes damage to healthy tissues.

In the current state of the art, the planning of ablation procedures, particularly those involving the use of ablation electrodes (a.k.a. needles or probes) for tissue ablation, is a complex and time-consuming process. This is due to the need for precise calculation of the trajectory for the ablation electrode, which must take into account a variety of factors, including the location and size of the tumor, the surrounding anatomy, and the specific constraints of the procedure. Traditional methods often rely on manual experience and the expertise of the medical practitioner, which can lead to variability in the outcomes and efficiency of the procedure. Furthermore, these methods do not typically allow for the fully-automatic execution and dynamic adjustment of the trajectory based on changing conditions during the procedure, which can limit their effectiveness and safety.

Therefore, an object of the present invention is to provide a solution which improves the drawbacks of prior art as mentioned above. In addition, the present invention aims to improve the clinical relevance of automatically calculated trajectories, which are feasible in the respective medical setting and scenario. Moreover, efficiency and accuracy and quality and safety of an ablation procedure should be improved.

The present invention addresses the above-mentioned disadvantages by providing a computer-implemented method, a trajectory calculator, and an ablation system as well as a computer program product for calculating a trajectory for an ablation electrode during an interventional procedure as claimed.

The method may involve receiving interventional image data, executing a segmentation algorithm on the data, determining target points and skin entry points, computing trajectory candidates, and executing an optimization algorithm to calculate the best trajectory. The system may include a trajectory calculator, an ablation tool, and potentially a user interface. The technology is designed to improve the precision and safety of interventional procedures such as tumor ablation.

As mentioned above, this method may involve the use of a segmentation algorithm to provide a set of segmentation masks, including a tumor mask and/or a body mask, based on received interventional image data. A set of target points and skin entry points are determined based on at least one of these masks, and a set of trajectory candidates is computed. The method may further involve the receipt of a set of pre-definable constraints, which are classified into mandatory and non-mandatory types. Differently sized and size-ordered subsets of trajectory candidates are defined, with these subsets differing in their conformity with the non-mandatory constraints. An optimization algorithm may then be executed on these subsets to calculate at least one trajectory, with the non-mandatory constraints being stepwise relaxed if no solution with 100% tumor coverage is found.

The key advantage of this invention is its ability to automate and optimize the calculation of the trajectory for the ablation electrode, thereby improving the efficiency and accuracy of the procedure. By using a segmentation algorithm to provide a set of segmentation masks, the method can accurately identify the location and size of the tumor and the surrounding anatomy. This allows for the precise calculation of the trajectory, taking into account the specific constraints of the procedure and the surgeon (e.g., to guide the instrument to a certain position).

In contrast to the existing literature, such as the algorithms described above, the methods and systems described in this invention disclosure depict technologies that are capable of delivering clinically applicable ablation needle plans by enabling fully-automatic thermal ablation planning and accounting for the needle positioning complexity.

In the context of this invention, the focus is on the planning and calculation of the trajectory for an ablation electrode. This involves the use of computer-implemented methods to process interventional image data, execute segmentation algorithms, determine target and skin entry points, and compute trajectory candidates. The method also involves the use of constraints, optimization algorithms, and potentially machine learning techniques to calculate the optimal trajectory for the ablation electrode.

Furthermore, the invention allows for the stepwise relaxation of non-mandatory constraints, which can increase the performance and the flexibility of the procedure and allow for the consideration of a wider range of trajectory candidates. This can improve the chances of finding a solution with 100% tumor coverage, thereby enhancing the effectiveness of the ablation.

In the following, the invention will be described with respect to the claimed method first. Features, advantages or alternative embodiments, mentioned in the context of the method can be assigned to the other claimed objects (e.g., the computer program or a device, in particular the trajectory calculator, the ablation system processing or a computer program product) and vice versa. In other words, the system, apparatus/device can be improved with features described or claimed in the context of the method and vice versa. In this case, the functional features of the method are embodied by structural units of the apparatus or device or system and vice versa, respectively. The method may refer to a software implementation and the device may refer to a hardware implementation. Generally, in computer science a software implementation and a corresponding hardware implementation (e.g., as an embedded system) are equivalent. Thus, for example, a method step for "storing" data may be performed with a storage unit and respective instructions to write data into the storage. For the sake of avoiding redundancy, although the device may also be used in the alternative embodiments described with reference to the method, these embodiments are not explicitly described again for the device. In principle, the respective trajectory calculator is configured to carry out the claimed method.

The present invention relates inter alia to a computer-implemented method for calculating a trajectory for an ablation electrode for use in an or during an interventional procedure, in particular for tissue ablation. The invention improves the efficiency, accuracy, and safety of the procedure by automating the calculation process and allowing for the dynamic adjustment of the trajectory based on changing conditions (like surgeon's and/or patient's movements).

Generally, ablation refers to a medical method, mainly executed percutaneously, used to inactivate, remove, destruct, cut away or kill abnormal cancerous and/or precancerous cells and/or cells, material, tissues, or layers, which induce cardiac arrhythmias. Ablation is executed by using ablation electrodes.

Ablation may be used as thermal ablation. In particular, Cryoablation may be applied, which is a type of cryotherapy, a minimally invasive interventional radiology procedure that uses extreme cold to freeze and kill abnormal cancerous cells or tissue. In addition or alternatively, Microwave Ablation, MWA, may be used, which uses microwave energy to generate heat for destroying targeted tissues.

Alternatively or in addition Radiofrequency Ablation, RFA, may be used, which uses heat generated by radio waves to destroy abnormal tissues.

Alternatively or in addition, laser ablation may be used, which is based on laser energy to remove or destroy abnormal tissues.

Alternatively or in addition, chemical ablation may be used, which is based on the injection of a chemical agent (e.g., alcohol or chemotherapy drugs) to destroy tissue, often used in liver or thyroid conditions.

Alternatively or in addition, electrochemical ablation may be applied, which uses electrical current to enhance the penetration of chemical agents into tissues for treatment.

Alternatively or in addition, High-Intensity Focused Ultrasound (HIFU) may be applied, which is based on sound waves to heat and destroy abnormal tissues.

In an example embodiment, the invention may be a microwave ablation for treating liver tumors.

According to a first aspect, the method may include several steps, starting with the reception of interventional image data. This data is then processed by executing a segmentation algorithm, which provides a set of segmentation masks. These masks include at least a tumor mask and a body mask.

The term ablation electrodes should be interpreted as means for executing or performing the ablation procedure, as mentioned above. The ablation electrode may be a needle, or probe. Thus the term ablation electrode should not only be construed as means being used for radiofrequency or microwave ablations, but also for cryoablation therapy or other forms of ablations, mentioned above.

A body mask may be or may comprise a mask (e.g., in the form of a visualized segmentation, highlighting and/or identification) of the skin. A body mask may be a representation of an outer shell or outside surface of the patient's body. Alternatively or in addition the body mask may be or comprise internal structures, in particular risk structures, which should not be impaired or damaged by the ablation insertion.

A tumor mask may be or may comprise a mask of the tissue, lesion or tumor to be processed by the ablation electrode. It may comprise the tumor in a segmented form. The tumor mask provides information, where the tumor is positioned within the patient's body, and/or its size and extensions in 3D space.

The method then may involve determining a set of target points based on the provided tumor mask, and a set of skin entry points in particular based on the provided body mask. Using these points, a set of trajectory candidates is computed, each candidate comprising at least a pair of target point and skin entry point.

The method may also include receiving a set of pre-definable constraints. These constraints are then classified into at least two classes: a first type constraint which are to be met mandatorily and a second type constraints which need not to be met mandatorily. The set of trajectory candidates all fulfil the first type constraints by definition.

The method may further involve defining differently sized and ordered subsets of trajectory candidates. These subsets differ in their conformity with the second type of constraints. An optimization algorithm is then executed on the defined subsets to calculate at least one trajectory as a result. This starts with an initial subset, which conforms with all first type constraints and with all second type constraints. If no solution with 100% tumor coverage is found, the optimization algorithm is iteratively executed for the next subset within the ordered subsets, so that the second type constraints are stepwise relaxed.

The segmentation algorithm provides segmented or extracted anatomical structures, which may be represented as masks. The mask has much less processing and storage requirements as they are a compressed and reduced representation of the respective anatomical region or organ or part of the original image. Segmentation may comprise partitioning an image into meaningful regions, such as tissues, organs, or abnormalities. Various algorithms are used depending on the imaging modality, the complexity of the anatomy, and the application, like pixel-intensity based approaches, edge-based methods, and/or model-based approaches, using prior context knowledge. For example, traditional segmentation algorithms with an active shape model or data driven model-based segmentation algorithms, which may be based on an image-to-image network may be applied. In an embodiment, for example a segmentation algorithm may be applied as disclosed in Active Shape Model: Heimann, Tobias, Ivo Wolf, and Hans-Peter Meinzer. "Active shape models for a fully automated 3D segmentation of the liver-an evaluation on clinical data." Medical Image Computing and Computer-Assisted Intervention-MICCAI 2006: 9th International Conference, Copenhagen, Denmark, October 1-6, 2006. Proceedings, Part II 9. Springer Berlin Heidelberg, 2006, or in Image-To-Image Network: Yang, Dong, et al. "Automatic liver segmentation using an adversarial image-to-image network." Medical Image Computing and Computer Assisted Intervention- MICCAI 2017: 20th International Conference, Quebec City, QC, Canada, September 11-13, 2017, Proceedings, Part III 20. Springer International Publishing, 2017.

Segmentation may provide the so-called masks. A mask generally identifies or characterizes the regions of interest (ROI) corresponding to targeted structures, like tumors, organs at risk or healthy tissue to be treated. Typically, the ROI relates to tumors. Therefore, a tumor mask is provided. However, it is also possible that the ROI relates to a non-tumor tissue. In this case, the "tumor" mask is adapted to segment the non-tumor ROI.

Different types of (segmentation) masks may be provided and processed, inter alia binary masks, which contains two-pixel values (e.g., 0 and 1). Pixels with a value of 1 represent the region of interest (e.g., a tumor), while pixels with a value of 0 represent the background or irrelevant regions or multi-class mask, which contain multiple values, where each value corresponds to a specific structure or class. For example: 0: Background, 1: Liver, 2: Kidney or vessels (organs of risk), 3: Tumor.

A mask comprises at least one tumor mask. The tumor mask may define or delineate the borders of a tumor. The tumor mask may be a 2D or 3D structure. Optionally, the segmentation masks may comprise relevant structure masks, defining the segmentation of relevant structures, comprising vessels, risky structures and/or problematic tissue. Representing and processing masks instead of the original image reduces processing requirements significantly and give an anatomical understanding of the anatomies, in particular a positional or geometrical information where the relevant anatomical structures are positioned in the patient's body. The tumor mask, represents the (delineations and /or borders and/or contours of) the tumor. The body mask represents the patient's body. Without body mask, it is not possible to determine skin entry points.

The step of defining differently sized ordered subsets of trajectory candidates may comprise generating subsets of different size, which differ in their fulfillment of the second type constraints. For example, an initial (or first) subset might be the smallest and complies with all first and second type constraints, e.g., with an axial constraint, a rib constraint and a long axis constraint. A second subset is larger as the initial subset and complies with all first and a first portion of second type constraints, e.g., with the axial constraint in addition with the rib constraint. A third subset is even larger as the second subset and complies with all first and a second portion of second type constraints, e.g., which may be the axial constraint as only constraint and so on. In a preferred embodiment, relaxation rules may be generated in a preparation procedure which comprise specification for the stepwise relaxing of second type constraints and/or how to generate subsets of trajectory candidates in a hierarchical order and how to apply them.

Preferably, the subsets (of trajectory candidates) are ordered according to the degree on how much they conform with the second type of constraints. The ordering of subsets may be such as initially starting with a small subset with all second type constraints (and first type constraints) and proceeding then to a second subset with a part of the second type constraints (and always the first type constraints) and so on.

Image data may be based on different modalities, comprising computed tomography (CT), x-ray imaging, Magnetic Resonance Imaging (MRI) and its variants like Functional MRI (fMRI), Diffusion Tensor Imaging (DTI), Ultrasound, nuclear medicine imaging systems, like Positron Emission Tomography (PET) and Single Photon Emission Computed Tomography (SPECT), Optical Coherence Tomography (OCT), and hybrid methods and others. The image data may be 2-, 3 or 4-dimensional data. The image data may be processed to provide segmentation masks, which are simplified representations of the anatomical structures or regions of interest, such as the tumor to be ablated and the surrounding tissues.

Preferably, the image data are interventional as they are acquired during the interventional ablation procedure. The patient is placed on the operating table and is imaged and is not moved until ablation procedure starts. Thus, no image registration needs to be carried out. Otherwise, image data may be received via an interface and image registration needs to be executed to map the received images to the ones acquired during or after the ablation procedure.

The set of trajectory candidates is a data structure with a pair of 3D positions. A trajectory candidate comprises at least one pair of target point (within or in the vicinity of the tumor) and assigned or associated skin entry point for the ablation needle, which both define the needle path and/or trajectory. Optionally, the trajectory candidate comprises an ablation protocol, defining at least ablation power and/or duration in addition. The set of trajectory candidates may be understood as a set of possible or candidate trajectories. However, the (final) result with the calculated trajectory is a subset of the trajectory candidates, namely the one which has been found to be the optimal solution in the given scenario (actual conditions, like patient data, anatomical data, tumor data, and/or ablation electrode data, feasibility for the surgeon etc.).

The term trajectory in this application may be interpreted as a (geometrical) line from skin entry point as starting point to target point in the tumor as endpoint, optionally with time indications, indicating how fast/slow the needle is proceeded in a certain path segment. The trajectory depends on the type of ablation electrode (length, power etc.) which may be provided as ablation dataset. The trajectory needs to be shorter than the length of the ablation electrode.

The trajectory together with the ablation electrode protocol exactly define at which position in space (3D), which energy is to be applied by the needle for how long.

The trajectory is influenced by various constraints, which can be classified into mandatory and optional constraints. These constraints can relate to clinical limitations, surgeon's instructions, and geometric parameters of the trajectory, among others.

The set of constraints is pre-definable in a preparation step. With this, the method may be adapted to different use cases (e.g., depending on the patient and/or clinical situation and/or surgeon etc.). The set of constraints is a data structure with one or more requirements or conditions, the trajectory has to fulfil. The constraints comprise mandatory and non-mandatory or optional constraints. Each trajectory has to meet the mandatory constraints (first type constraints) and should map with the optional ones (second type constraints) as much as possible. A mandatory constraint may be a clinical limitation (like 100% tumor coverage with safety margins) and/or surgeon's instructions (like limitations for skin entry point). The optional ones may be ranked according to priority and are processed according to the assigned priority. Preferably, the ranking may be amended by user input on a user interface. The set of constraints may relate to limitations or requirements for the trajectory of the ablation electrode, which may be provided in the form of a needle.

The constraints, in particular the second type constraints may relate to the trajectory and/or may refer to at least one of the following geometric parameters:
- the trajectory length;
- its longitudinal direction, in particular with respect to the longitudinal axis of the tumor (or its geometric longest diameter);
- a rib related measure, which defines the needle trajectory with respect to the position of at least one rib, in particular, defining, that the needle passes through an intercostal space such that it is close a pre-definable upper margin with one of the two surrounding ribs, in particular with the lower rib;
- an angulation measure, which defines how many planes need to be used for adjusting the needle's trajectory. For example, the constraint may be defined to at least use two planes for adjusting the needle's trajectory, like e.g., axial and transversal plane.

In addition or alternatively, the constraint may relate to an ablation-related parameter:
- a power measure, defining an amount or upper limit of ablation power or energy, in particular to avoid over ablation (excess or surplus energy with regard to the aim to only destroy non-healthy tissue) etc.
- a time measure, e.g., an ablation duration, e.g. in the form of an upper limit for how long the ablation procedure should last or be;
- Energy type of ablation, like radiofrequency, RF, or microwave, irreversible electroporation (IRE), laser, etc.... and/or
- Number of needles, e.g., maximum number of needles.

In an embodiment, it may be possible to calculate an interim result. The interim result may comprise the indication or suggestion which energy type should be used for the ablation (see examples, mentioned above, RF or microwave etc. The suggestion is determined algorithmically and may be based on tumor entity, tumor size, tumor geometry and/or tumor location.

The step of iteratively executing the optimization algorithm for the next subset has the technical effect of stepwise relaxing the second type constraints, which may comprise deleting certain constraints from the set of constraints. This progressive relaxation of constraints leads to larger search space for the optimization algorithm.

The complexity algorithm may initially start with a first subset of trajectory candidates, e.g. those conforming with three constraints A, B and C, wherein A, B and C are placeholders for any of the defined second type constraints, in particular those, mentioned above (like the trajectory length; its longitudinal direction, in particular with respect to the longitudinal axis of the tumor, a rib related measure, and/or an angulation measure). In the second iteration the subset is enlarged, comprising trajectory candidates conforming with constraints is A+B and in the next iteration A+C. In the next iteration only A may be used as constraint for executing the optimization algorithm. The initial subset usually conforms with all first and second type constraints. The next subset in the order of subsets, e.g., a second subset conforms with all first type constraints and with a first part of the second type constraints. The next subset in the order of subsets, e.g., a third subset conforms with all first type constraints and with a second part of the second type constraints and so on, so that the search space for the optimization algorithm is enlarged with each iteration, preferably until a solution is found or until the maximum number of ablation electrodes is reached without finding a solution.

The result comprises the at least one calculated trajectory for one ablation electrode. Alternatively, it may comprise in addition indications for performing the ablation, like time indications (duration of the ablation), power settings for the ablation electrode and/or indications for the surgeon.

The method may be re-iterated for further ablation electrodes, so that a plurality of ablation electrode trajectories is defined. In particular, the method is executed to find a single needle solution. If no such solution is found, the number of needles is increased.

The invention also involves the use of optimization algorithms to calculate the optimal trajectory based on the defined constraints. This process can involve iterative steps and the progressive relaxation of optional constraints to expand the search space for the optimization algorithm. The result of this process is a calculated trajectory for the ablation electrode, which can then be used to guide the actual ablation procedure.

The use of an optimization algorithm also allows for the selection of the trajectory that best conforms with the pre-definable constraints and/or requirements and for reducing the complexity to find a solution for a multi-constraint multi-objective combinatorial optimization problem and even for dynamic adjustment of the trajectory based on changing conditions during the procedure, enhancing the effectiveness and safety of the ablation.

The step of iteratively executing the optimization algorithm for the next subset has the technical effect of stepwise relaxing the second type constraints, which may comprise deleting certain constraints from the set of constraints. This progressive relaxation of constraints leads to larger search space for the optimization algorithm.

In an embodiment of the method, the first type constraints include the requirement for 100% tumor coverage. This means that the trajectory for the ablation electrode should be calculated in such a way that the entire tumor can be ablated. This is a mandatory requirement that must be met in order for the trajectory to be considered viable.

Alternatively or in addition to this, the first type constraints may also include the requirement that risky tissue structures and/or healthy tissue should not be harmed. This means that the trajectory for the ablation electrode should be calculated in such a way that it avoids these structures. This is also a mandatory requirement that must be met in order for the trajectory to be considered viable.

These first type constraints are crucial in ensuring that the ablation procedure is both effective in treating the tumor and safe for the patient. By ensuring 100% tumor coverage, the method ensures that the entire tumor can be treated. By avoiding risky tissue structures and healthy tissue, the method minimizes the risk of complications and side effects from the procedure.

Alternatively or in addition, the first type constraints may include surgeon's instructions. These instructions can be received from user input via a user interface. This allows the surgeon or other medical professional to provide specific instructions or requirements for the trajectory of the ablation electrode. For example, the surgeon may specify certain areas of the tissue that should be avoided, or they may specify a particular approach angle for the electrode. These instructions can then be incorporated into the calculation of the trajectory, ensuring that the resulting trajectory aligns with the surgeon's specific requirements or preferences.

This feature provides a high degree of flexibility and customization in the calculation of the trajectory. It allows the method to be tailored to the specific needs and preferences of the individual surgeon, and to the specific circumstances of the individual patient and tumor. This can help to improve the effectiveness and safety of the ablation procedure.

Alternatively or in addition, the second type constraints may include one or more geometric parameters. These geometric parameters can provide additional guidance for the calculation of the trajectory for the ablation electrode.

One such parameter is an axial constraint, which specifies that the ablation electrode should be in the plane of the axial image slice in which the tumor mask is represented, or at least as close as possible to this plane. This can help to ensure that the electrode is properly aligned with the tumor on the basis of the interventional image taken.

Another parameter is a rib constraint, which specifies that the ablation electrode should pass through an intercostal space. In particular, the electrode should be close to the upper margin of one of the surrounding ribs, particularly the lower rib. This constraint can help to make the trajectory clinically feasible as intercostal vessels are not damaged and advantageously uses the ribs as a support structure for the needle.

A further parameter is a long axis constraint, which specifies that the longitudinal axis of the ablation electrode should be parallel to the longest diameter of the tumor. This can help to ensure that the electrode is properly positioned to treat the entire tumor efficiently.

Yet another parameter is a double-angulated constraint, which specifies that the ablation electrode needs to be adjusted or defined in two different planes and/or does not need match the axial constraint. This can provide additional flexibility in the positioning of the electrode, allowing it to be adjusted to best suit the specific circumstances of the tumor and surrounding tissue.

These second type constraints provide additional guidance for the calculation of the trajectory, helping to ensure that the resulting trajectory is both effective for treating the tumor and safe for the patient. However, as they are second type constraints, they need not be met mandatorily, providing additional flexibility in the calculation of the trajectory.

In another embodiment of the method, the step of determining the set of target points is based not only on the provided tumor mask, but also on ablation data. This ablation data can be received via an ablation interface and can define parameters and/or functionality of the ablation electrode.

The ablation data define parameters and/or functionality of the ablation electrode (as different products for ablation electrode exist). In particular, the ablation data can include information such as the power of the electrode and/or the duration of the ablation. This information can be used to calculate the coverage of the ablation, i.e., the area of tissue that will be affected by the ablation procedure. By taking this information into account when determining the set of target points, the method can ensure that the calculated trajectory for the ablation electrode will allow for effective treatment of the entire tumor.

This feature can help to improve the effectiveness of the ablation procedure, by ensuring that the entire tumor is covered by the ablation. It can also help to improve the safety of the procedure, by ensuring that the trajectory for the electrode is calculated in such a way as to avoid damaging healthy tissue or risky structures.

In another embodiment of the method, the step of determining a set of skin entry points involves executing a path tracing algorithm. This algorithm evaluates virtual paths from the target point to potential skin entry points, ensuring that these paths do not intersect with risky or impassable structures.

Risky or impassable structures could include vital organs or blood vessels that could be damaged during the ablation procedure. Impassable structures could include artificial structures within, on, or around the patient's body, such as anatomical structures, such as bones and/or cartilages and/or stents and/or implants, and/or metal structures that could interfere with the ablation electrode.

By using a path tracing algorithm to determine the set of skin entry points, the method can ensure that the calculated trajectory for the ablation electrode avoids these risky or impassable structures. This can help to improve the safety of the ablation procedure, by minimizing the risk of damage to vital structures within the patient's body.

In another embodiment of the method, the step of computing a set of trajectory candidates involves executing a selection algorithm. This algorithm is configured to select feasible trajectory candidates that meet a set of requirements. Requirements may be user-defined. The set of requirements may be comprised in the set of constraints. Alternatively, the set of requirements may be stored and processed separately from the set of constraints.

The selection algorithm serves to improve performance by reducing the search space for the optimization algorithm. It does this by only determining those trajectory candidates that meet the requirements. This step can be interpreted as a filtering step that filters out unfeasible candidates.

For instance, candidates that touch or intersect risky anatomical structures, would cause irreparable tissue damage, or are difficult for the medical practitioner to execute may be filtered out. This ensures that the optimization algorithm only considers trajectory candidates that are both feasible and safe, improving the efficiency of the method and the safety of the resulting ablation procedure.

In another embodiment of the method, the result, which represents the calculated trajectory, is used for image-based guidance during the ablation procedure. This can involve comparing the actual path of the ablation electrode with the calculated trajectory. If there are deviations between the actual path and the calculated trajectory, a warning signal may be output. This can alert the medical practitioner to the deviation, allowing them to adjust the path of the electrode as necessary. The warning signal may be provided in different format, like optic, acoustic and/or vibration signals.

In addition to or instead of this, other guidance options may be used. For example, a laser could be pointed at the patient's body to show the entry point and the direction in which the clinician should insert the needle according to the calculated trajectory. This procedure could be repeated for each of the needle candidates found.

Alternatively, a robot could perform the needle insertion entirely without human assistance. In this case, the trajectory and entry point from the calculated result could be automatically transferred to the robot. This could help to ensure precise and accurate placement of the ablation electrode, improving the effectiveness and safety of the ablation procedure.

In another embodiment of the method, executing the optimization algorithm involves executing a reinforcement learning (RL) algorithm. This RL algorithm is used to train a RL agent for optimally placing and/or guiding an ablation electrode.

Reinforcement learning is a type of machine learning where an agent learns to make decisions by taking actions in an environment to achieve a goal. The agent learns from the consequences of its actions, rather than from being explicitly taught, adjusting its behavior based on the positive or negative feedback it receives.

In the context of the invention, the RL agent could be trained to determine the optimal trajectory for the ablation electrode based on the constraints and the data from the segmentation masks. The RL agent could then use this learned knowledge to guide the placement of the ablation electrode during the actual ablation procedure.

This use of reinforcement learning can help to improve the effectiveness and efficiency of the method. By learning from past experiences, the RL agent can make more informed decisions about the placement of the ablation electrode, potentially leading to more effective and safer ablation procedures.

In another embodiment of the method, multiple different reinforcement learning (RL) agents are trained for optimizing each iterative step for the stepwise relaxation of the constraints. These RL agents are trained on different subsets of constraints.

The training of the RL agents is executed prior to any use in the clinical environment. Training of the RL agents may comprise using a population of retrospective clinical data and large amounts of augmentation. This can include placing the tumor in arbitrary positions of the organ, applying different rotations and/or scalings, and/or even elastic deformations. This leads to the possibility to generate virtually infinite data from an existing cohort.

Since the training operates on segmentation masks, it is independent of the image modality or acquisition. The training is specific to the constraint subset and for a specific ablation electrode configuration. An ablation electrode configuration may refer to an electrode type and/or the expected ablation zone chart, i.e. expected ablation size subject to power setting and/or ablation duration and/or tissue type. The electrode configuration may alternatively or in addition comprise the electrode count, but the network could also learn to identify the optimal number of electrodes by adding or removing electrodes.

Once trained, the RL agents are applied for a new scenario or patient after the image acquisition and before performing the intervention. This use of multiple RL agents, each trained on a different subset of constraints, can help to improve the efficiency and effectiveness of the method. By using RL agents that are specifically trained for each subset of constraints, the method can more effectively optimize the trajectory for the ablation electrode, potentially leading to more effective and safer ablation procedures.

For more details on the reinforcement learning process, reference can be made to the work of Chaitanya, Krishna, et al. in "Automatic planning of liver tumor thermal ablation using deep reinforcement learning," presented at the International Conference on Medical Imaging with Deep Learning, and to patent application US 2024 011 532 0 A1.

In another aspect, the present invention also relates to a trajectory calculator for calculating a trajectory for an ablation electrode for tissue ablation during an interventional procedure. The trajectory calculator comprises several components, including an image interface for receiving interventional image data and a segmentation unit. The segmentation unit is configured to execute a segmentation algorithm on the received interventional image data to provide a set of segmentation masks, which includes at least a tumor mask and a body mask.

The trajectory calculator also includes a processing unit. This processing unit is configured to determine a set of target points based on the provided tumor mask and to determine a set of skin entry points in particular based on the provided body mask. The processing unit is also configured to compute a set of trajectory candidates, where each candidate comprises at least a pair of target point and skin entry point.

The trajectory calculator further includes a constraint interface for receiving a set of pre-definable constraints and a classifier. The classifier is configured to classify the received set of constraints into at least two classes: a first type constraint which are to be met mandatorily and a second type constraints which need not to be met mandatorily.

The processing unit is further adapted to define differently sized and ordered subsets of trajectory candidates, where the subsets differ in conforming with the second type of constraints. The trajectory calculator also includes an optimizer. The optimizer is configured to execute an optimization algorithm on the defined subsets to calculate at least one trajectory as a result. This starts with an initial subset, which conforms with all first type constraints and with all second type constraints. If no solution with 100% tumor coverage is found, the optimizer is configured to iteratively execute the optimization algorithm for the next subset within the ordered subsets, so that the second type constraints are stepwise relaxed.

Finally, the trajectory calculator includes an output interface for providing the result with the calculated at least one trajectory. This output can then be used to guide the placement of the ablation electrode during the interventional procedure.

In another embodiment, the trajectory calculator is further configured to perform any of the steps outlined in the previous method as described above. This includes, but is not limited to, receiving and classifying constraints, determining target and skin entry points, computing trajectory candidates, and executing an optimization algorithm.

The trajectory calculator may also include additional features as described previously in relation to the method. For example, it may be configured to use reinforcement learning algorithms, consider surgeon's instructions, or account for geometric parameters and ablation data in its calculations.

This flexibility allows the trajectory calculator to be adapted to a wide range of scenarios and requirements, improving its utility and effectiveness in calculating trajectories for ablation electrodes during interventional procedures.

In another aspect, the present invention also encompasses an ablation system for executing an ablation procedure for an object, particularly a patient. This system comprises a trajectory calculator as described above, and an ablation tool with a set of ablation electrodes.

The trajectory calculator is responsible for calculating the optimal trajectory for the ablation electrodes during the procedure. It does this by processing interventional image data, executing a segmentation algorithm to provide a set of segmentation masks, determining target points and skin entry points, computing trajectory candidates, and executing an optimization algorithm to calculate the final trajectory.

The ablation tool, on the other hand, is equipped with a set of ablation electrodes, which are controllable. The position and/or movement control of the ablation electrodes may preferably be executed on the basis of the calculated trajectory. Thus, these electrodes are used to perform the actual ablation procedure, following the trajectory calculated by the trajectory calculator.

This ablation system combines the advanced trajectory calculation capabilities of the trajectory calculator with the practical ablation capabilities of the ablation tool and/or its user (interventional radiologist). This allows for more precise and effective ablation procedures, potentially leading to better patient outcomes.

In another embodiment, the ablation system includes a user interface. This user interface can be used for the input of constraints, for defining the cost function, and/or for defining a set of requirements for the selection algorithm.

The user interface allows for direct input from the user, such as a medical professional. This can include inputting specific constraints that the trajectory must meet, defining the cost function that the optimization algorithm will use, and/or defining the set of requirements that the selection algorithm will use to select feasible trajectory candidates.

This feature provides a high degree of flexibility and customization in the calculation of the trajectory. It allows the ablation system to be tailored to the specific needs and preferences of the individual user, and to the specific circumstances of the individual patient and tumor. This can help to improve the effectiveness and safety of the ablation procedure.

The present invention also includes a computer program product. This product comprises program elements which, when loaded into a memory of a computing device, cause the device to perform the steps of the method as described above.

The program elements can include various types of software code, such as source code, object code, or executable code. These program elements can be stored on a computer-readable medium, such as a hard drive, solid state drive, optical disc, flash drive, or any other type of storage device.

When these program elements are loaded into the memory of a computing device, they cause the device to perform the various steps of the method. This includes receiving interventional image data, executing a segmentation algorithm, determining target points and skin entry points, computing trajectory candidates, receiving and classifying constraints, defining subsets of trajectory candidates, and executing an optimization algorithm to calculate a trajectory.

This computer program product allows the method to be implemented on a computing device, such as a computer, server, or other type of processing device. This can provide a convenient and efficient way to calculate trajectories for ablation electrodes during interventional procedures.

In another aspect the invention relates to a computer program, the computer program being loadable into a memory unit of a computing unit, including program code sections to make the computing unit execute the method for calculating a trajectory as described above, when the computer program is executed in said computing unit.

In another aspect the invention relates to a computer-readable medium, on which program code sections of a computer program are stored or saved, said program code sections being loadable into and/or executable in a computing unit to make the computing unit execute the method for calculating a trajectory as described above, when the program code sections are executed in the computing unit.

### Short description of the Figures

- Fig. 1: is a flow chart of a method for calculating an ablation trajectory according to an embodiment of the present invention;
- Fig. 2: is a block diagram of a trajectory calculator according to an embodiment of the present invention;
- Fig. 3: is a schematic representation of an ablation system for executing an ablation procedure according to optional embodiments of the invention and
- Fig. 4: is an illustration of a possible user interface of the method.

### Detailed description

Cancer is the second leading cause of death worldwide and can affect almost any tissue type and organ (e.g., liver, lung, kidney, and bone) [1]. One promising therapeutic option is thermal ablation, which aims at inducing tumor necrosis via thermal damage by inserting percutaneously one or more ablation needles. Treatment success of thermal ablation is commonly achieved if the tumor as well as a safety margin (e.g., greater than 5 mm for liver tumors) is ablated [2,3], which mitigates risks of local tumor recurrence. It is therefore particularly important to precisely plan and/or execute the interventional procedure.

In general, planning thermal ablation procedures is extremely challenging and time-consuming. In essence, it comprises of finding the optimal number of ablation needles and their optimal skin entry and target points, as well as the optimal ablation configuration (e.g., power and duration in microwave ablation), while accounting for 3D coverage of irregularly shaped tumors, the large number of surrounding organs at risk, and additional organ-specific physiological and pathophysiological constraints that may alter the ablation zone (e.g., the cooling effect (heat sink) of major vessels in the liver). In addition, there are several soft constraints one should account for, e.g., the trajectory length, distances to risk structures, etc. Since it may not be possible to find a single solution that is optimal with respect to all hard and soft constraints, planning may therefore consider finding a Pareto optimal solution.

Another problematic aspect is that the availability of supporting technology (e.g., laser guidance system) may vary significantly between individual operators and clinical sites, so that there exists a large variation of how thermal ablation is being performed.

In state of the art, computer-aided methods have the drawbacks that clinical constraints are typically not considered sufficiently and that they are not fully automated and thus require significant manual user input.

The invention described herein presents a novel method and system to enable fully automatic thermal ablation planning, which accounts for the complexity of the ablation needle placement and which can be triggered with a single button click.

The ablation electrodes or needles used may vary in their function, depending on the manufacturer's settings (e.g., power and other parameters, indicative for example for ablative margin). These settings are algorithmically considered when calculating the ablation needle path.

In an embodiment, the calculated and predicted path (trajectory) may be visualized. In particular, the calculated path may be visualized against the actual needle placement or path continuously during the interventional procedure. This may help the medical practitioner to perform the future placements of the needle.

In another embodiment, deviations from the calculated trajectory to the executed (actual) trajectory may be provided to a quality assessment algorithm which is configured to judge the quality of the algorithmic and automatic trajectory calculation. In case quality is judged to be not sufficient, in particular in case of deviations between the actually executed trajectory and the calculated trajectory and in case the actually executed trajectory was judged to be better, an improvement dataset may be stored. This improvement dataset may be provided to an optimization algorithm and/or to the method for calculating the trajectory and may serve as a feedback signal in a feedback loop for continuously improving the method, based on real cases.

The following paragraphs explain two different embodiments for algorithms for fully-automatic one-click planning, which considers the ablation needle placement complexity into the optimization process. In both scenarios, the availability of medical images (MRI, CT, US, etc.) to characterize both the tumor as well as other medically relevant structures (risk structures like internal organs, skeletal bones, etc.) is a pre-requisite. Specifically, the medical images may be preprocessed and segmented to have clean segmentation masks of all relevant structures available. The availability of an accurate tumor segmentation, however, is essential.

In addition, a method to compute expected ablation zones is required. In one embodiment this amounts to computing geometrical primitives subject to the device manufacturer's ablation zone sizing charts. In another particularly advantageous embodiment, this may comprise having access to a biophysical model capable of accurately forecasting the necrosis zone based on the patient physiology, information about the ablation device, the range of possible power settings, and the ablation duration(s).

### A) First embodiment based on Conventional Optimization:

This paragraph describes a deterministic algorithm for fully automatic thermal planning that follows a deterministic algorithm, and which does not train a neural network. This algorithm may, however, expose a set of control parameters that could be fine-tuned based on a cohort of synthetic data and/or clinical patient data. Without lack of generality, one advantageous embodiment of a conventional planning algorithm may comprise the following steps:

### 1. Treatment Zone Definition

The suggested pipeline will define the treatment zone as the tumor + safety margin. In one embodiment, the safety margin may be adjustable by the operator. To this end, the tumor segmentation is dilated by the specified margin value. In another embodiment and to achieve full automaticity, the safety margin may be factored into the optimization process subject to a range constraint. In Layman's terms the algorithm may aim to find a solution that balances the amount of additional healthy tissue destroyed, but which destroys a minimum of 5mm to reduce chances of local recurrence and not more than another adjustable parameter. To enable full automaticity, one can tune and fix this upper threshold parameter based on synthetic and/or patient data.

### 2. Definition of Suitable Needle Candidates

Next, the set of needle candidates for the conventional optimization is computed. To this end, suitable target points (=needle tip inside the body) and suitable skin entry points must be computed.

Target Points: In one embodiment, this step may comprise defining all points inside and in the near vicinity of the treatment zone. In another advantageous embodiment, the set of target points may be constrained based on prior knowledge about the procedure. For instance, for microwave ablation the needle is commonly pushed through the tumor due to the ellipsoidal ablation zone shape. This may translate to searching for target points distal to possible skin entry points, i.e., points at the back and lateral side of the treatment zone and within a certain distance to the treatment zone surface.

Skin Entry Points: In one embodiment, the set of skin entry points may be extracted from the segmentation masks by querying all points on the skin surface. To increase computational performance, the set of points may be subsampled to a set of equidistant points. In another embodiment, the set of skin entry points may be computed by applying or executing a path tracing algorithm. This path tracing algorithm is based on "shooting" rays from each candidate target point and finding the intersection with the torso (patient's) surface.

Needle Candidate Selection: Not all pairs of skin entry and target points are valid needle candidates, because a specific needle trajectory may not be executable if it could injure a critical structure, if a bony structure overlaps with the trajectory, or if it cannot reach the end point (e.g., because the end point exceeds the needle length). For the latter, candidates may be filtered out by comparing the virtual needle trajectory against the information about the maximum needle length of the ablation device. To compute the risk of injuring a critical structure, ray tracing and intersection testing may comprise one particularly advantageous embodiment.

In another embodiment, this may be accomplished by training a neural network on raw medical images (see preceding invention disclosure on segmentation free ablation planning) to perform the intersection testing.

### 3. Conventional Optimization Accounting for Needle Positioning Complexity

Given the set of valid needle candidates, the final step comprises the actual planning algorithm, i.e., finding one ablation needle candidate or a set of ablation needle candidates that achieves optimal results subject to a set of hard and soft constraints and which accounts for the complexity of executing/positioning the needle(s).

In prior art where algorithms do not consider complexity consideration, this step would comprise in testing each needle candidate, needle candidate combinations, or applying combinatorial optimization solvers to find the solution to the planning problem which leads to bad performance. Candidate testing may comprise computing one or more ablation zones subject to a set of possible ablation configurations (e.g., ablation zone sizes reflecting different power settings and ablation durations in microwave or radiofrequency ablation), their treatment zone coverage, volume of affected healthy tissue, distances to risk structures, etc.

To account for the complexity of ablation needle positioning and the clinical feasibility, to improve performance (reduce the planning time), and to enable full automaticity, the proposed algorithm tests candidates of disjoint and progressively larger and more complex solution spaces. Without lack of generality, one particularly advantageous embodiment may denote a deterministic algorithm that could be extended and fine-tuned with large amounts of clinical expert knowledge. The proposed algorithm would start by searching for a single needle in - particularly disjoint - solutions spaces ranked from simplest to most difficult to perform by a clinician, for example:
1. Axial & On-The-Rib & Long Axis
2. Axial & Long Axis
3. Axial & On-The-Rib
4. Axial
5. On-The-Rib & Long Axis
6. On-The-Rib
7. Double-Angulated & Long Axis
8. Double-Angulated

That is, first only candidates are tested that are axial (=transversal, i.e, in-plane to the image slices showing the treatment zone), have a needle trajectory sitting on top of the rib (not through the ribs), and that are aligned with the tumor long axis (#1 or first solution space). Testing may comprise testing one or more ablation zone configurations and computing a cost value for each configuration candidate based on a cost function that reflects one or more constraints to be optimized (e.g., the trajectory length, the amount of over ablation, etc.). If there exists no solution achieving 100% coverage, solution space #2 would be tested, i.e., all solutions that are axial, aligned with the long axis, and which have not been tested in #1, are tested. Similarly, if there is no optimal solution for space #2, the next more complex space would be tested (i.e., #3). If after processing #1 to #8 there is no single needle solution, the number of needles would be increased and the solutions spaces are tested again in the same ordering from simple to complex.

To increase the performance of the algorithm, one may store (locally on a hard drive or in RAM, alternatively in a cloud storage solution) the ablation zone configuration(s) per ablation needle candidate during the first testing run of the subproblem spaces #1 to #8. For any consecutive run that tests for solutions with more than one ablation needle, one may load the previously computed subspace ablation configurations and apply a multi-constraint multi-objective combinatorial optimization routine onto the previously computed configurations. In one embodiment, this may comprise of finding multi-needle solutions that do not need to have the same ablation configuration (i.e., each ablation needle can have a different power setting and ablation duration). In another particularly advantageous embodiment, this may comprise finding only multi-ablation-needle solutions that have the same ablation configuration, which may be easier to perform for the clinician and which may reduce the compute time, because less candidates need to be tested.

The process may be repeated until a solution is found or the maximum number of needles is reached without finding a solution. In the former case, one must differentiate between two scenarios:
(1) A single solution achieving 100% coverage of the treatment zone was found: Consequently, this solution is the optimal solution and it will be returned to the operator, because it is the easiest feasible solution achieving 100% coverage of the treatment zone.
(2) More than one ablation needle configuration achieving 100% coverage of the treatment zone were found: In one embodiment, one may return the solution with highest or lowest cost (additive cost when considering multiple needles). In the case that more than one configuration has the highest or lowest (additive) cost value, one may apply a different weighting of the multiple objectives (e.g., increasing the weight associated with the optimization of the trajectory length or the extent of over ablation) and return the solution with highest/lowest new cost value.

In another advantageous embodiment, the complexity consideration could be added to any existing planning algorithm with code access by adding to or multiplying the cost function with a complexity weighting of candidates. One may define the complexity weighting either deterministically, e.g., by calibrating a hashing function to clinical data or feedback from clinician.

Alternatively or in addition (e.g., for verification purposes), one may train a neural network to map the medical images (or segmentation), the needle trajectories, and other relevant information (such as the ablation configuration) to a complexity score. This network may then be trained with large amounts of synthetic or clinical data. Alternatively, one may use large amounts of clinical feedback to train the system.

### B) Second embodiment with Al-Based Planning:

The following paragraphs describe an embodiment of Al-based planning, extending technologies described in previous invention disclosures. Specifically, thermal ablation planning is framed as a reinforcement learning problem, where a neural agent is automatically learning to optimally place the ablation needle(s) by interacting with a virtual patient environment.

During training, the network would explore different needle placements by changing the needle position(s), i.e., changing the skin entry or target point or, alternatively, changing one end point and the needle direction. After each move, the network would receive either a positive or negative reward, respectively depending on whether it optimizes the cost function or disobeys the hard constraints.

There are several options how to adapt such Al-based methods to account for the needle placement complexity.
(1) In one embodiment, multiple different agents are trained to optimize a specific solution space subset, e.g., using the same subspace ordering of the total solution space as defined in the conventional optimization section, and for different needle configurations (ablation zone sizes and number of needles).
   During inference, one would apply the same approach as with the conventional optimization routine. In brief, first neural agents for single needle planning are applied and agents from simple to complex are executed sequentially. If no single solution is found, more needles are tested, while accounting for the complexity ordering of the neural agents. Since each neural agent is extremely fast and efficient to execute, it may be possible to execute multiple neural agents of multiple subproblems in parallel. If any solution exists among the set of subproblems, one may return the optimal solution that is the simplest to execute.
(2) In another embodiment, one may adapt the reward function to account for the complexity of needle placement(s). Specifically, one may derive a deterministic cost function that reflects the complexity. One exemplary cost function may be realized by a function where 2 denotes the simplest solution and 5 the most complex and which scales the reward function, i.e., the reward is significantly increased for simple solutions and reduced for very complex solutions. Other definitions may also be feasible and can be fine-tuned based on the availability of large amounts of synthetic and/or clinical patient data.

Alternatively, one may train a neural network on synthetic, clinical patient data, or based on clinical feedback (e.g., by having a clinician sort various solutions) to estimate the complexity of a needle plan (see one embodiment at the end of the conventional planning algorithm description. With any of such complexity descriptors one may then either train neural agents from scratch or, alternatively, re-fine existing neural agents to account for the needle placement complexity.

The novel system described herein allows for fully automatic planning of thermal ablation procedures that prefers simple needle trajectories and that is triggered by a single button click. Specifically, this invention disclosure describes two variants: (1) a deterministic algorithm that is based on conventional optimization routines; and (2) an Al-based approach.

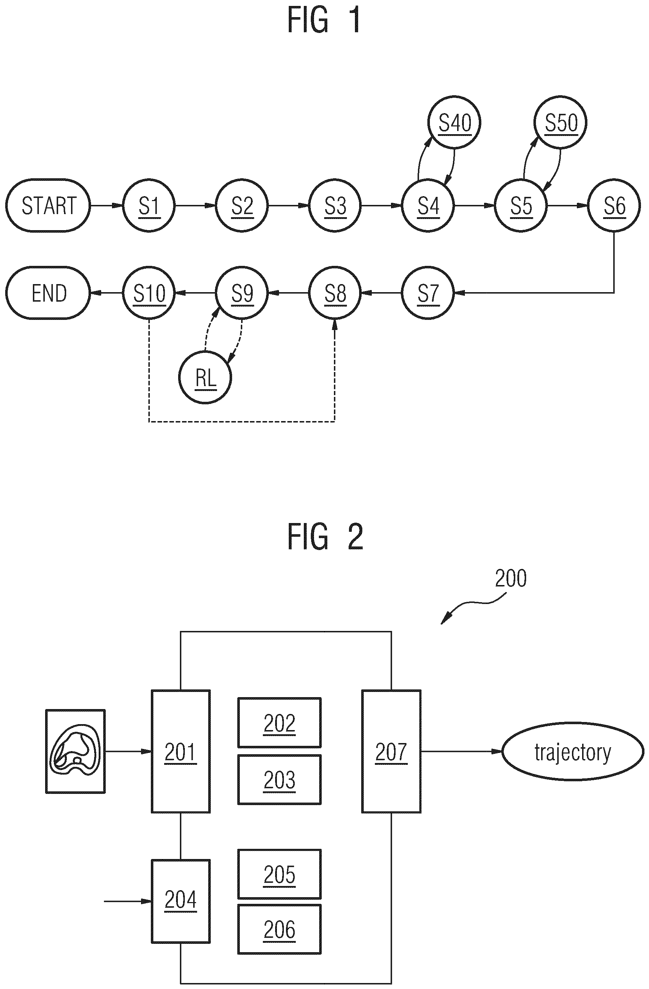

Fig. 1 shows a flowchart of a method for calculating a trajectory for an ablation electrode for tissue ablation during an interventional procedure according to possible embodiments. The method may comprise receiving interventional image data, like CT an/or MRI in step S1. In step S2 a segmentation algorithm is executed on the received interventional image data for providing a set of segmentation masks, comprising at least a tumor mask and a body mask. In step S3 a set of target points is determined, based on the provided tumor mask. In step S4 a set of skin entry points is determined, based on the provided body mask. In step S5 a set of trajectory candidates is calculated, wherein a trajectory candidate comprises at least a pair of target point and skin entry point. In step S6 a set of pre-definable constraints is received. The constraints may be defined or selected on a user interface III by the user. In step S7 the received set of constraints is classified in at least two classes: a first type constraint which are to be met mandatorily and a second type constraints which need not to be met mandatorily. In step S8 differently sized and ordered subsets of trajectory candidates are defined, wherein the subsets differ in conforming with the second type of constraints. In a further step an optimization algorithm S9 is executed on the defined subsets for calculating at least one trajectory as result in step S10, starting with an initial subset, conforming with all first type constraints and with all second type constraints by computing a cost value, based on a cost function, and if no solution with 100% tumor coverage is found: iteratively executing the optimization algorithm S9 for the next subset within the ordered subsets, so that the second type constraints are stepwise relaxed. The iteration may be represented in Fig. 1 with a dotted line from step S10 to step S8.

As depicted in Fig. 1 in dotted lines, the step S4 of determining the skin entry points may optionally comprise executing a path tracing algorithm S40. The path tracing algorithm S40 defines rays or paths, defined by each candidate target point and an intersection point, intersecting or interacting with the torso (patient's) surface.

Alternatively or in addition, the step S5 of computing a set of trajectory candidates may optionally comprise executing a selection algorithm S50. The selection algorithm S50 is configured to select those trajectory candidates which are feasible and that meet a set of requirements. The selection algorithm thus may be configured for determining those trajectory candidates that meet the requirements. This step can be interpreted as a filtering step that filters out unfeasible candidates. Requirements may be comprised in the set of pre-definable constraints and/or may be user defined in particular for the respective interventional procedure (patient, surgeon etc.).

Alternatively or in addition, the step of executing the optimization algorithm S9 may optionally comprise a reinforcement learning algorithm RL.

Generally, it is possible to execute the steps in another sequence, where appropriate. For example, the sequence of steps S3 and S4 may be exchanged.

The method may be re-iterated for each trajectory planning and/or execution. In particular, the method for calculating the trajectory as a result may be executed online and/or during the interventional ablation procedure.

Fig. 2 represents a trajectory calculator 200, which is configured to receive inter alia interventional image data and provide a calculated trajectory as result. In particular, the trajectory calculator 200 for calculating a trajectory for an ablation electrode for tissue ablation during an interventional procedure may comprise several modules or physical entities.

The trajectory calculator 200 may comprise an image interface 201 for receiving interventional image data.

The trajectory calculator 200 may comprise a segmentation unit 202, which is configured for executing a segmentation algorithm on the received interventional image data for providing a set of segmentation masks, comprising at least a tumor mask and a body mask.

The trajectory calculator 200 may comprise a processing unit 203, configured for determining a set of target points, based on the provided tumor mask and for determining a set of skin entry points, based on the provided body mask and for computing a set of trajectory candidates, wherein a trajectory candidate comprises at least a pair of target point and skin entry point.

The trajectory calculator 200 may comprise a constraint interface 204 for receiving a set of pre-definable constraints. This may be implemented as user interface UI, so that the user has flexibility of defining certain constraints.

The trajectory calculator 200 may comprise a classifier 205, configured for classifying the received set of constraints in at least two classes: a first type constraint which are to be met mandatorily and a second type constraints which need not to be met mandatorily.

The processing unit 203 is adapted for defining differently sized and ordered subsets of trajectory candidates, wherein the subsets differ in conforming with the second type of constraints.

The trajectory calculator 200 may comprise an optimizer 206, configured for executing an optimization algorithm on the defined subsets for calculating at least one trajectory as result, starting with an initial subset, conforming with all first type constraints and with all second type constraints by computing a cost value, based on a cost function, and if no solution with 100% tumor coverage is found: iteratively executing the optimization algorithm for the next subset within the ordered subsets, so that the second type constraints are stepwise relaxed. The optimizer 206 may be implemented or integrated in the processing unit 203.

The trajectory calculator 200 may comprise an output interface 207 for providing the result with the calculated at least one trajectory.

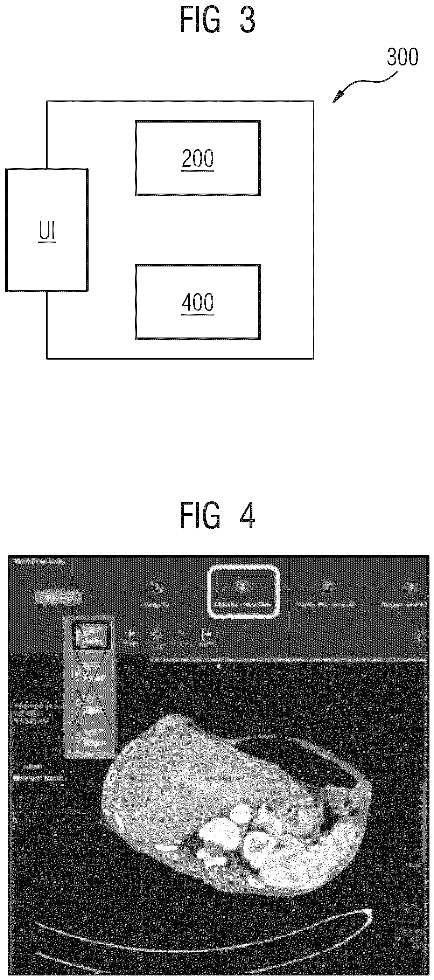

Fig. 3 schematically represents an ablation system 300 which comprises the trajectory calculator 200 and an ablation tool 400, which may be a medical device for executing the ablation procedure. As depicted in Fig. 3 with dotted lines, the ablation system optionally may comprise a user interface UI, which may be explained in more detail with respect to Fig. 4.

Fig. 4 shows an example of a possible user interface UI. In contrast to existing and preceding planning algorithms (which require pressing specific buttons; crossed out options in graphic), the fully automatic planning algorithm may be triggered by pressing a single button - here: "Auto" and the trajectory calculator automatically calculates a trajectory which conforms with the received constraints and/or requirements.

## Claims

1. Computer-implemented method for calculating a trajectory for an ablation electrode for tissue ablation during an interventional procedure, comprising the following method steps:
- Receiving (S1) interventional image data;
- Executing a segmentation algorithm (S2) on the received interventional image data for providing a set of segmentation masks, comprising at least a tumor mask and a body mask;
- Determining (S3) a set of target points, based on the provided tumor mask;
- Determining (S4) a set of skin entry points, based on the provided body mask;
- Computing (S5) a set of trajectory candidates, wherein a trajectory candidate comprises at least a pair of target point and skin entry point;
- Receiving (S6) a set of pre-definable constraints;
- Classifying (S7) the received set of constraints in at least two classes: a first type constraint which are to be met mandatorily and a second type constraints which need not to be met mandatorily;
- Defining (S8) differently sized and ordered subsets of trajectory candidates, wherein the subsets differ in conforming with the second type of constraints,
- Executing an optimization algorithm (S9) on the defined subsets for calculating (S10) at least one trajectory as result, starting with an initial subset, conforming with all first type constraints and with all second type constraints by computing a cost value, based on a cost function, and if no solution with 100% tumor coverage is found: iteratively executing the optimization algorithm for the next subset within the ordered subsets, so that the second type constraints are stepwise relaxed.

2. Method according to claim 1, wherein the first type constraints comprise 100% tumor coverage, while not harming risky tissue structures and/or damaging healthy tissue.

3. Method according to any of the preceding claims, wherein the first type constraints comprise surgeon's instructions, which are received from user input via a user interface (UI).

4. Method according to any of the preceding claims, wherein the second type constraints comprise at least one of the following geometric parameters:
- An axial constraint, defining that the ablation electrode needs to be in a plane of the axial image slice, in which the tumor mask in represented or at least as close as possible to this plane;
- A rib constraint, defining that the ablation electrode passes through an intercoastal space, in particular that the ablation electrode is close to an upper margin of one of the surrounding ribs, in particular of the lower rib;
- A long axis constraint, defining that a longitudinal axis of the ablation electrode is parallel to the longest diameter of the tumor and/or
- A double-angulated constraint, defining that the ablation electrode needs to be adjusted in two different planes and/or does not match the axial constraint.

5. Method according to any of the preceding claims, wherein the step of determining (S3) the set of target points is based on the provided tumor mask and ablation data, wherein the ablation data are received via an ablation interface.

6. Method according to any of the preceding claims, wherein the step of determining (S4) a set of skin entry points comprises executing a path tracing algorithm (S40) which evaluates virtual paths from the target point to skin entry points and assures not to intersect with risky or impassable structures.

7. Method according to any of the preceding claims, wherein the step of computing (S5) a set of trajectory candidates comprises executing a selection algorithm (S50), configured to select feasible trajectory candidates meeting a set of requirements.

8. Method according to any of the preceding claims, wherein the result is used for image-based guidance during the ablation procedure.

9. Method according to any of the preceding claims, wherein executing the optimization algorithm (S9) comprises executing a reinforcement learning algorithm (RL) for training a RL agent for optimally placing and/or guiding an ablation electrode.

10. Method according to the directly preceding claim, wherein multiple different agents are trained for optimizing each iterative step for the stepwise relaxation of the constraints.

11. Trajectory calculator (200) for calculating a trajectory for an ablation electrode for tissue ablation during an interventional procedure, comprising:
- An image interface (201) for receiving interventional image data;
- A segmentation unit (202), which is configured for executing a segmentation algorithm on the received interventional image data for providing a set of segmentation masks, comprising at least a tumor mask and a body mask;
- A processing unit (203), configured for determining a set of target points, based on the provided tumor mask and for determining a set of skin entry points, based on the provided body mask and for computing a set of trajectory candidates, wherein a trajectory candidate comprises at least a pair of target point and skin entry point;
- A constraint interface (204) for receiving a set of pre-definable constraints;
- A classifier (205), configured for classifying the received set of constraints in at least two classes: a first type constraint which are to be met mandatorily and a second type constraints which need not to be met mandatorily;
- wherein the processing unit (203) is adapted for defining differently sized and ordered subsets of trajectory candidates, wherein the subsets differ in conforming with the second type of constraints,
- An optimizer (206), configured for executing an optimization algorithm on the defined subsets for calculating at least one trajectory as result, starting with an initial subset, conforming with all first type constraints and with all second type constraints by computing a cost value, based on a cost function, and if no solution with 100% tumor coverage is found: iteratively executing the optimization algorithm for the next subset within the ordered subsets, so that the second type constraints are stepwise relaxed
- An output interface (207) for providing the result with the calculated at least one trajectory.

12. Trajectory calculator (200) according to the immediately preceding claim, further configured to perform any of the steps of any of the method claims 2 to 10 and/or having any of the features of any of the method claims.

13. Ablation system (300) for executing an ablation procedure for an object, in particular a patient, comprising:
- A trajectory calculator (200) according to claim 11 or 12 and
- An ablation tool (400) with a set of ablation electrodes.

14. Ablation system (300) according to claim 13, wherein the ablation system (300) further comprises a user interface (UI) for input of constraints, for defining the cost function, and/or for defining a set of requirements for the selection algorithm.

15. Computer program product comprising program elements which cause a computing device to perform the steps of the method for calculating a trajectory for an ablation electrode for tissue ablation during an interventional procedure according to any of the preceding method claims when the program elements are loaded into a memory of the computing device.
